# EUROPÄISCHE PATENTSCHRIFT

(11) **EP 2 583 632 B1**
(45) Veröffentlichungstag und Bekanntmachung des Hinweises auf die Patenterteilung: **09.05.2018**
(21) Anmeldenummer: 12199263.0
(22) Anmeldetag: 05.06.2003
(51) Int. Cl.: A61B 17/16, A61B 17/17, A61C 1/08, A61C 8/00, A61F 2/30

(54) **Implantat zum partiellen Ersatz von belasteten Flächen**
Implant for partial replacement of loaded surfaces
Implant destinés à remplacer, en partie, des surfaces chargées

(30) Priorität: 06.06.2002 DE 10225217
(43) Veröffentlichungstag der Anmeldung: 24.04.2013
(62) Teilanmeldung aus: 03756969.6
(73) Patentinhaber: medArtis Medizinprodukte und Forschung AG, 81545 München (DE)
(72) Erfinder: Draenert, Klaus, 81545 München (DE)
(74) Vertreter: Vossius & Partner Patentanwälte Rechtsanwälte mbB

(56) Entgegenhaltungen:
- WO-A-01/32109
- WO-A1-01/80772
- DE-A- 2 953 575
- US-A- 3 879 767
- US-A- 4 784 661
- US-A- 6 039 568
- US-B1- 6 284 002
- US-B1- 6 315 795

## Beschreibung

Die vorliegende Erfindung betrifft eine Anordnung zum insbesondere partiellen Ersatz von belasteten Flächen, wie z.b. Gelenkflächen. Sie kann jedoch auch zum Ersatz von Kauflächen tragenden Kieferbereichen samt Zähnen oder Stiften verwendet werden.

Bislang ist es üblich, bei einem Verschleiß von Gelenkflächen das gesamte Gelenk oder Gelenkteile in toto durch eine Endoprothese zu ersetzen.

Es ist Ziel der vorliegenden Erfindung, Implantate und eine Anordnung zu schaffen, die einen partiellen Ersatz von belasteten Flächen wie Kau- oder Gelenkflächen ermöglicht, ohne den Einbau einer Endoprothese in toto zu erfordern. Diese Aufgabe wird erfindungsgemäß durch Implantate nach Anspruch 1 gelöst. Vorteilhafte Weiterbildungen der Erfindung sind Gegenstand der zugeordneten Unteransprüche.

Wenn nachfolgend von Gelenkflächen die Rede ist, so schließt dies auch andere belastete Flächen, wie z.B. die Kauflächen eines Zahnes mit ein. Der Grundkörper wird in einen ausgenommenen Bereich eines Knochens eingesetzt, der zuvor aus dem Knochen z.b. durch Bohren, Stanzen, Fräsen, mittels Laser oder durch andere vergleichbare Abtragungstechniken abgetragen worden ist. Der Grundkörper wird vorzugsweise mit Preßsitz in dem abgetragenen Bereich gehalten, obwohl auch andere Befestigungen mit Friktionssitz oder Formschluß, z.b. Gewinde vorstellbar sind. Der Grundkörper ist als Zylinder ausgebildet sein und trägt direkt die Gelenkfläche. Die erfindungsgemäßen Implantate bestehen vorzugsweise aus einem zylindrischen Grundkörper mit zwei einander abgewandten Stirnseiten, von denen die erste Stirnseite oder die Peripherie des Grundkörpers eine vorzugsweise in einem Winkel ungleich 90 Grad zur Zylinderachse verlaufende gerade oder gekrümmte Fläche ausweist, die die zu ersetzende Gelenkfläche bildet, und die zweite Stirnseite ebenfalls eine vorzugsweise in einem Winkel ungleich 90 Grad zur Zylinderachse verlaufende gerade oder gekrümmte Fläche ausweist, die nach der Implantation mit der Knochenperipherie abschließt. Statt der Fläche kann der Körper auch einen Adapter zur Aufnahme eines die zu ersetzende Fläche enthaltenden Flächenelements z.B. Gelenkflächenelements oder Zahns aufweisen. Der Winkel der Fläche(n) kann jedoch auch 90° betragen.

Die die Gelenkflächen sind vorzugsweise oberflächenbehandelt, um so in ihrer Oberflächenstruktur die natürliche Gelenkflächenstruktur möglichst detailgetreu nachzubilden. Mit dem Knochen oder dem umgebenden Gewebe in Kontakt tretende Teile des Implantats können in an sich bekannter Weise mit Antibiotika beschichtet und/oder bestückt sein, um Entzündungen zu vermeiden. Ebenso können Knochenersatzwerkstoffe aus einem entsprechend behandelten Material hergestellt, z.b. geformt oder gesintert sein.

Das Implantat passt vorzugsweise derart in den ausgestanzten bzw. ausgebohrten Knochenbereich, dass es im Preßsitz gehalten wird. Der Zylinderdurchmesser des Implantats ist dann vorzusweise minimal, z.B. 0,01 bis 0,5 mm größer als das Bohrloch im Knochen. Die Tatsache, dass die auf die Gelenkfläche wirkenden Kräfte nicht axial in das Implantat sondern in einem gewissen Winkel eingeleitet werden, ist für den Preßsitz vorteilhaft. Selbstverständlich kann der Zylindermantel des Implantats jedoch auch aufgerauht bzw. mit hintergreifenden Strukturen, z.B. Gewinde versehen sein, was jedoch bezüglich der Traumatisierung des umgebenden Knochengewebes von Nachteil ist.

Vorzugsweise ist das Implantat einstückig, d.h. in einem Herstellungsvorgang aus einem Material hergestellt, was die Herstellung vereinfacht. Es kann jedoch auch vorgesehen sein, daß der zylindrische Grundkörper und die zweite Stirnseite aus einem herkömmlichen Knochenersatzwerkstoff bestehen, während die erste Stirnseite, die die Gelenkfläche bildet, durch ein spezielles Gelenkflächenmaterial wie z.B. spezielle Titanlegierungen oder Keramik gebildet ist. Wahlweise können auch beide Stirnseiten aus anderem Material bestehen als der zylindrische Implantatgrundkörper.

Falls der Grundkörper einen Adapter für das Flächenelement aufweist, kann der Grundkörper in seiner Funktion als Knochenersatzwerkstoff optimiert sein, während das aufgenommene Flächenelement als Gelenkflächenelement optimiert sein kann. Die Materialwahl ist entsprechend abzustimmen. Als Adapterkombination eignen sich alle bekannten Verbindungstechniken mit Gewinde oder anderen Fomrschlußtechniken, Reibschluss oder Preßsitz.

Die Form des Implantats oder der Gelenkflächen z.B. an wenigstens einer der beiden Stirnseiten kann in an sich bekannten Formnachbildungsverfahren dem ausgebohrten bzw. ausgestanzten Knochenteil nachgebildet werden, wobei darauf zu achten ist, dass der Durchmesser des Grundkörpers derart vergrößert wird, dass der Implantatkörper im Preßsitz in dem Knochen gehalten ist.

Zur Herstellung des Implantats eignen sich alle gängigen Implantatwerkstoffe und Knochenersatzwerkstoffe.

Zur Durchführung der Implantation umfasst eine erfindungsgemäße Anordnung vorzugsweise eine Winkelbestimmungsanordnung in Form mehrerer Ellipsenplatten. Die Form der Ellipsenplatten entspricht der Schnittfläche durch einen Kreiszylinder unter einem bestimmten Schnittwinkel x relativ zur Ebene quer zur Zylinderachse. Je größer der Schnittwinkel ist, desto länger ist die Ellipse. Die Breite der Ellipse wird erhalten durch den Durchmesser des Kreiszylinders. Es werden Ellipsenplatten vorgesehen mit vorzugsweise unterschiedlichem Durchmesser als auch unterschiedlichem Schneidwinkel. Der Sinn der unterschiedlichen Geometrie dieser Ellipsenplatten liegt darin, die für einen Defekt passende Ellipsenplatte derart auszuwählen, dass sie zwar den defekten Gelenkflächenbereich vollständig überdeckt, gleichzeitig aber möglichst wenig intakte Gelenkfläche. Wenn man die korrekte Ellipsenplatte herausgefunden hat, gibt der Schnittwinkel der Ellipsenplatte exakt den Bohrwinkel und den Bohrdurchmesser für das zu entnehmende und zu ersetzende Knochenmaterial an, wobei die Bohrung in dem entsprechenden Winkel und mit dem entsprechenden Durchmesser derart gesetzt wird, dass die Bohrung in dem defekten Gelenkflächenbereich endet.

Um eine entsprechende Substitution des Knochenmaterial zu ermöglichen, umfasst die erfindungsgemäße Anordnung ferner ein Winkelführungsgerät, das einen Fixierfortsatz zur Einführung zwischen den Gelenkflächen hat. Dieser Fixierfortsatz kann beispielsweise die Ellipsenplatte der Winkelbestimmungsanordnung sein oder jeder andere Fortsatz, der eine Fixierung des Winkelführungsgerätes zwischen den Gelenkflächen ermöglicht. Mit dem Fixierfortsatz ist ein Winkelstellgerät verbunden, das eine Führung bzw. Bohrführung, die relativ zum Fixierfortsatz entweder in dem von der Ellipsenplatte angegebenen Winkel verläuft oder deren Winkel auf den von der Ellipsenplatte angegebenen Schneidwinkel einstellbar ist.

Die Führung an dem Winkelstellgerät ist in dem eingestellten Winkel relativ zum Fixierfortsatz angeordnet und ermöglicht das Setzen eines Führungsdrahtes im Operationsgebiet zur Befestigung einer Bohrführung, die es ermöglicht, einen Bohrer in dem voreingestellten Winkel durch den Knochen zu führen, so dass er exakt in der zu ersetzenden Gelenkfläche endet. Nach dem Setzen des Führungsdrahtes wird die Bohrführung in dem durch den Führungsdraht vorgegebenen Winkel und Abstand von der defekten Gelenkfläche befestigt, und anschließend wird der Zylinderbohrer zum Abtragen des Knochenmaterials durch diese Bohrführung in den abzutragenden Knochenbereich geführt.

Anstelle der Führung zum Setzen des Führungsdrahtes kann auch die Bohrführung direkt an dem Winkelstellgerät vorgesehen sein, in welchem Fall der Zylinderbohrer zum Abtragen des Knochenmaterials durch das Winkelstellgerät selbst in den Knochen geführt wird.

Eine Bohranordnung zum Abtragen des Knochenmaterials bzw. zum Bohren des Durchganges enthält einen Bohrer bzw. eine Stanze, der/die durch die Bohrführung führbar ist. Die Bohrführung ist entweder durch die Führungsdrähte oder das Winkelstellgerät selbst in einer festen Winkellage relativ zu dem restaurierenden Gelenkflächenabschnitt derart gehalten, dass die Bohrachse im Zentrum des zu ersetzenden Gelenkflächenabschnitts endet.

Zur Erzeugung eines sauberen Schnitts, der exakt in der Ebene der Gelenkfläche endet, enthält die Bohranordnung vorzugsweise ein Oszillationsbohrgerät, das einen Hohlzylinderbohrer mit abgeschrägter Spitze aufweist, wobei die Abschrägung der Spitze exakt dem Bohrwinkel des Bohrers relativ zur Gelenkfläche entspricht. Der Oszillationswinkel des Oszillationsbohrgerätes ist ausgesprochen klein, vorzugsweise weniger als 10 °, insbesondere weniger als 5 °. Auf diese Weise wird erreicht, dass der Bohrer durch das Bohrgerät bzw. die Bohrmaschine direkt bis an die Gelenkfläche geführt werden kann, wobei die abgeschrägte Spitze genau parallel zur Gelenkfläche liegt, so dass keine Kante des Bohrers bereits in die gegenüberliegende Gelenkfläche einschneidet, wenn die gegenüberliegende Bohrerkante gerade die Gelenkfläche erreicht hat.

Vorzugsweise sind die Zylinderbohrer selbst als dünnwandige hohlzylindrische Bohrer ausgebildet, deren abgeschrägte Spitze eine harte Schleifkrone aufweist, die insbesondere mit einem Schleifstein oder Hartmetallbesatz, insbesondere mit einem Diamantbesatz versehen ist, so dass die Schärfe des Bohrers auch nach oftmaliger Anwendung gewährleistet bleibt und das Knochengewebe möglichst wenig traumatisiert ist.

Die Erfindung wird nachfolgend beispielsweise anhand der schematischen Zeichnung beschrieben. In dieser zeigen:
- Fig. 1: einen Satz von Ellipsenplatten zur Bestimmung der Bohrgröße und des Bohrwinkels,
- Fig. 2: ein Winkelführungsgerät, dessen Fixierfortsatz durch eine Ellipsenplatte gebildet ist,
- Fig. 3: ein Winkelführungsgerät, bei dem der Fixierfortsatz durch einen Fixierdom für Kniegelenke gebildet ist,
- Fig. 4: eine Bohrführung,
- Fig. 5: eine Bohrführung mit Zylinderbohrer,
- Fig. 6: eine zweite perspektivische Ansicht der Bohrführung mit Zylinderbohrer aus Figur 5,
- Fig. 7: zwei Zylinderbohrer mit abgeschrägter Spitze in Seitenansicht,
- Fig. 8: Zylinderbohrer mit abgeschrägter Spitze zum Handbetrieb,
- Fig. 9: eine schematische Ansicht des Vortriebs des Zylinderbohrers mit abgeschrägter Spitze im Operationsgebiet,
- Fig. 10/11: eine schematische Darstellung des Ausstanzens und Ersetzen eines defekten Gelenkflächenbereichs, und
- Fig. 12: eine perspektivische Ansicht zweier in einem Femur implantierter Implantate zur Restauration von Kniegelenksabschnitten .
- Fig. 13-16: weitere Ausführungsformen erfindungsgemäßer Implantate.

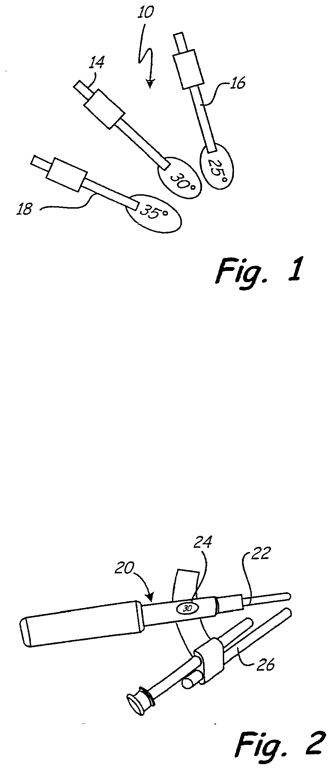

Figur 1 zeigt drei Ellipsenplatten, welche durch einen schrägen Schnitt aus einem Kreiszylinder entstehen. Die Winkelabgabe aus den Ellipsenplatten geben dabei den Schnittwinkel relativ zu einer Querschnittsfläche zur Zylinderachse an. Dementsprechend bedingt eine große Winkelabweichung vom Zylinderquerschnitt eine entsprechend längere Ellipsenform und eine geringere Abweichung eine entsprechend kürzere Länge der Ellipse. Die Breite der Ellipse entspricht dem Zylinderdurchmesser. Der Satz an vorzusehenden Ellipsenplatten umfasst sowohl unterschiedliche Winkel als auch unterschiedliche Durchmesser, so dass alle Größen von Gelenkflächendefekten mit den Ellipsenplatten abgedeckt werden können. Die Ellipsenplatten werden derart über der defekten Gelenkstelle angeordnet, dass die defekte Gelenkstelle vollflächig überdeckt wird, zugleich aber möglichst wenig gesunde Gelenkfläche. Der Winkel und die Breite, d.h. der Durchmesser der Ellipsenplatte legen den Bohrwinkel und den Durchmesser des Bohrers fest, der zum Abtragen von Knochenmaterial verwendet wird. Der Knochenabtrag erfolgt hierbei in dem von der Ellipsenplatte angegebenen Winkel relativ zur Gelenkfläche, wobei der ausgebohrte bzw. ausgestanzte Zylinderbereich in der defekten Gelenkfläche mündet. Dieser ausgebohrte bzw. ausgeschnittene oder ausgestanzte Knochenbereich wird später durch ein Knochenersatzmaterial auf natürlicher Basis wie z. B. Hydroxylapatit oder durch keramische oder metallische Implantatmaterialien z. B. auf Titanbasis ersetzt, wobei das in die Gelenkfläche ragende Ende des Knochenersatzmaterials dann die ausgeschnittene defekte Gelenkfläche substituiert.

Anstelle der Ellipsenplatten ist es jedoch auch möglich, durch die Messung der Länge und der Breite der Defektstelle den korrekten Schneidwinkel und Bohrdurchmesser für das abzutragende Knochenmaterial zu bestimmen.

Ferner umfasst die Anordnung zur partiellen Gelenkflächenrestauration ein Winkelführungsgerät, das es ermöglicht, entweder den Bohrer in dem gewünschten Winkel relativ zur Gelenkfläche durch den Knochen zu führen oder zum Setzen von Führungsdrähten, an welchen eine Bohrführung befestigt werden kann, die dann die Führungsaufgabe für den Bohrer in entsprechender Weise wahrnimmt. Das Bohrloch wird so gesetzt, dass es in dem vorher bestimmten Abstand und Winkel durch den Knochen führt und in der Defektstelle endet, womit durch den Bohrvorgang die Gelenkflächendefektstelle aus dem Knochen entfernt wird.

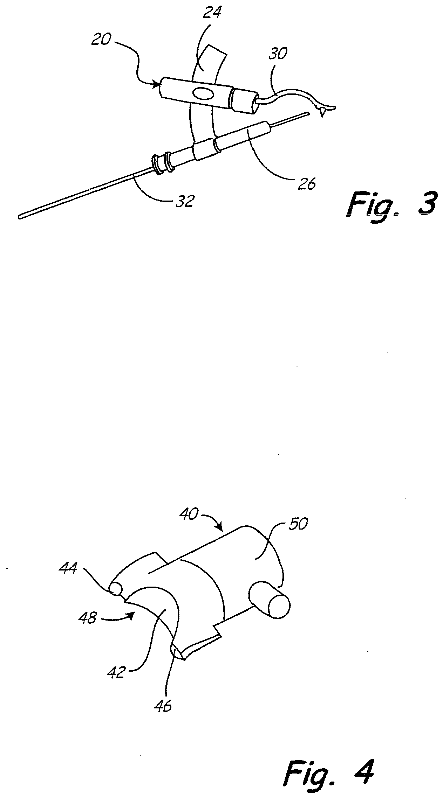

Hierfür hat das Winkelführungsgerät 20 einen Fixierfortsatz 22, der in diesem Fall durch eine Ellipsenplatten aus Figur 1 gebildet ist, einen Winkelstellmechanismus 24 und eine Führung 26 zum Setzen von Führungsdrähten in dem Knochen. Zusätzlich hat das Winkelstellgerät 20 zur besseren Handhabung an seinem hinteren Ende einen Handgriff 28. Der Fixierfortsatz 22 ist vorzugsweise lösbar in dem Winkelstellgerät 20 gehalten, so dass unterschiedliche Fixierfortsätze in das Winkelstellgerät 20 einsetzbar sind, wie dies beispielweise in Figur 3 gezeigt ist, bei welcher der Fixierfortsatz 30 durch einen Fixierdorn zum Einsatz in das Kniegelenk gebildet ist. Die Figur 3 zeigt zudem einen Führungsdraht 32, der durch die Führung 26 in dem Knochen gesetzt wird, um eine Bohrführung an dem Knochen zu fixieren, die beispielsweise in Figur 4 dargestellt ist.

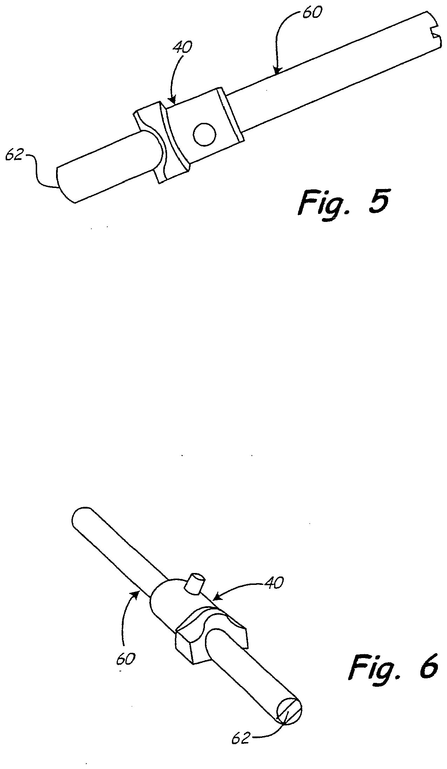

Die Bohrführung 40 umfasst einen Anlagebereich 42 zur Anlage an den Knochen und zwei Führungslöcher 44, 46, die von Führungsdrähten 32 durchsetzbar sind. Die Führungsdrähte sind z.B. mittels des Winkelstellgerätes 20 der Figuren 2 und 3 im Knochen gesetzt worden. Die Bohrführung 40 ist insgesamt als Hohlzylinder ausgebildet mit einem inneren Zylinderbereich 48, in welchem ein Bohrer gemäß den Figuren 5 bis 8 geführt wird. Die Bohrführung 40 umfasst ferner eine Drehhülse 50, die innenseitig Spannelemente (nicht dargestellt) aufweist, um einen geführten Bohrer in einer definierten Position zu fixieren.

Figur 5 zeigt eine Bohrführung 40 zusammen mit einem Zylinderbohrer 60, der eine Diamantkrone 62 aufweist. Der Zylinderbohrer 60 ist sehr dünnwandig und innen hohl, so dass das Knochenmaterial nur im Mantelbereich des Zylinderbohrers 60 geschnitten wird, während das abzutragende Material in dem Innenhohlraum des Bohrers verbleibt. Die Traumatisierung des Gewebes ist somit minimal.

Figur 6 zeigt die gleiche Anordnung mit Bohrführung 40 und Zylinderbohrer 60 von schräg vorne.

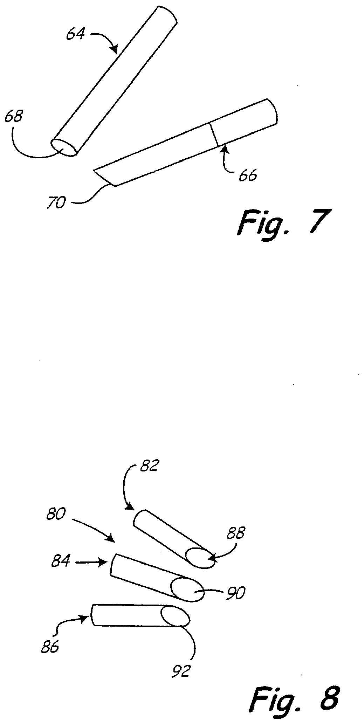

Wenn der Knochen in einem schrägen Winkel angebohrt werden soll, wird vorzugsweise eine Oszillationsbohreinrichtung mit einem geringen Oszillationswinkel von maximal 10 ° insbesondere maximal 5 ° verwendet. In diesem Fall ist die Spitze der Zylinderbohrer 64, 66 vorzugsweise abgeschrägt 68, 70, so dass die durch die Schräge gebildete Ellipsenfläche des Bohrerkopfes am Ende des Bohrvorganges genau in der Gelenkebene endet, wodurch sichergestellt wird, dass beim Abtragen, d. h. Ausstanzen des Knochenmaterials der Bohrer mit seiner Kante nicht in das Gewebe zwischen den Gelenkflächen oder in die gegenüberliegende intakte Gelenkfläche einschneidet. Durch den geringen Oszillationswinkel im Zusammenhang mit der dem Schneidwinkel angepassten Schräge des Zylinderbohrers wird somit sichergestellt, dass das vordere Ende 68, 70 der Bohrer exakt in der Gelenkebene endet. Sollte das ausgestanzte Material dennoch an ein paar Fasern hängen, so ist vorzugsweise ein Satz 80 von Handbohrern 82 bis 86 (Fig. 8) vorgesehen, deren schräge Spitze 88 bis 92 exakt der Schrägfläche der Bohrer 64, 66 aus Figur 7 und exakt der Ellipsenform der Ellipsenplatten aus Figur 1 entspricht. Durch die manuellen Bohrer 82 bis 86 kann somit das ausgestanzte Knochenmaterial schonend aus dem Operationsgebiet gelöst werden.

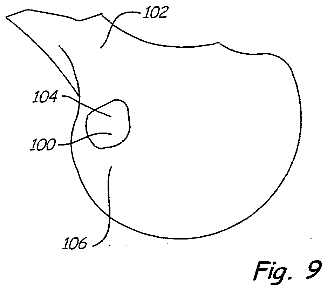

Nach dem Herausbohren und Herausnehmen des Knochenmaterials wird dann, wie es in Figur 9 gezeigt ist, ein Implantat 100 in den Knochen 102 eingesetzt, wobei das Material des Implantats an der gelenkseitigen Stirnfläche 104 und an der Außenseite des Knochens 106 oberflächenbearbeitet wird, um zum einen die Gelenkfläche in Form und Glätte möglichst naturgetreu nachzubilden und zum anderen die Außenfläche des Knochens zur Vermeidung einer Traumatisierung des umgebenden Gewebes möglichst genau nachzubilden.

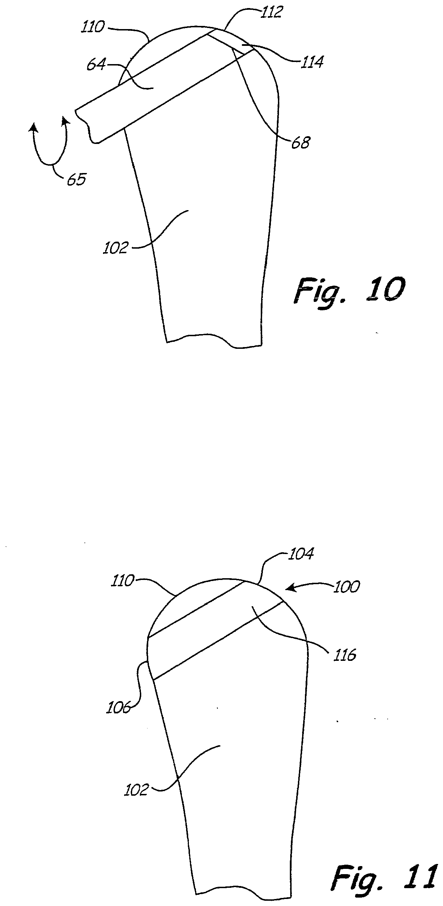

Der Vorgang des Ausbohrens mit der Oszillationsbohrvorrichtung und des Anschließenden Substituierens des ausgefrästen Knochenbereichs mit einem Implantat ist schematisch in den Figuren 10 und 11 wiedergegeben.

Fig. 10 zeigt einen Knochen 102 mit einer oberen Gelenkfläche 110 , z.B. entsprechend dem Kugelgelenk des Femurs. Mit einem Hohlzylinderbohrer 64 aus Fig. 7 mit abgeschrägter Spitze 68 wird nun das abzutragende Knochenmaterial unter dem defekten Gelenkflächenbereich 112 ausgestanzt. Durch die Verwendung einer Oszillationsbohrvorrichtung 65 mit geringem Oszillationswinkel von etwa 5 Winkelgrad bleibt die Schräge 68 in etwa parallel zur Oberfläche des defekten Gelenkflächenabschnitts 112. Wenn nun die schräge Krone 68 der Hohlzylinderstanze 64 die Oberfläche 112 erreicht, liegt sie ziemlich genau in der Ebene des defekten Abschnitts 112 und traumatisiert umgebendes Gewebe des Gelenks nur minimal.

Gemäß Fig. 11 wird nach dem Herausnehmen des zu ersetzenden Knochenmaterials 114 die dadurch entstandene zylindrische Bohrung im Knochen 102 durch ein Implantat 100 gefüllt, wie es z.B. in Fig. 9 bereits gezeigt wurde.

Das Implantat umfasst einen zylindrischen Grundkörper 116, dessen abgewandte Stirnseiten 104 und 106 den defekten Gelenkflächenbereich 112 und die Knochenperipherie substituieren. Die zum Gelenk weisende Stirnseite 104 des Implantats 100 wird oberflächenbehandelt, um die Geometrie und Oberflächenbeschaffenheit des umgebenden Gelenkflächenbereichs 110 so gut wie möglich nachzubilden. Es sind auch an sich bekannte Formnachbildungsverfahren anwendbar, um die Stirnseite 104 der Gelenkfläche aus dem Gelenkflächenbereich 112 so gut wie möglich nachzubilden. Auch die zur Knochenperipherie weisende Stirnseite 106 des Implantats 100 wird oberflächenbehandelt, um einen homogenen und gewebeverträglichen Abschluss mit der umgebenden Knochenoberfläche zu erzeugen.

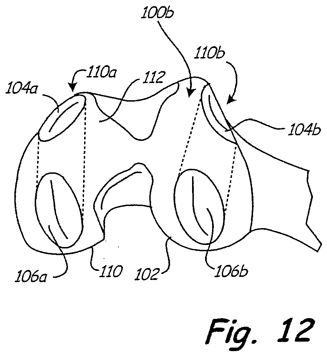

Fig. 12 zeigt zwei Implantate 100a, 100b, die in einem Kniegelenk eines Femurknochens 102 implantiert sind. Die Implantate 100a,b bestehen aus einer Titanlegierung, die für Gelenkimplantate üblich ist. Die ersten Stirnseiten 104a,b der Implantate bilden einen Teil der Gelenkfläche 112 und sind derart oberflächenbehandelt, dass die fluchtend mit der umgebenden Gelenkfläche 112 das Gelenk wieder in seiner ursprünglichen Funktionalität wiederherstellen. Die Implantate sitzen im Preßsitz in den im Knochen 102 ausgefrästen Löchern, womit sie gegen axiale Verschiebung auch bei starker Beanspruchung des Gelenks festgelegt sind. Hierbei ist anzumerken, dass durch die Tatsache, dass die Stirnflächen 104a,b der Implantate 100a,b relativ zur Zylinderachse des Grundkörpers 106b in einem Winkel ungleich 90 Grad geneigt sind, zu einer nicht axialen Krafteinleitung auf das Implantat führt. In der Figur ist sehr schön der zylindrische Grundkörper 106b des rechten Implantats 100b zu sehen.

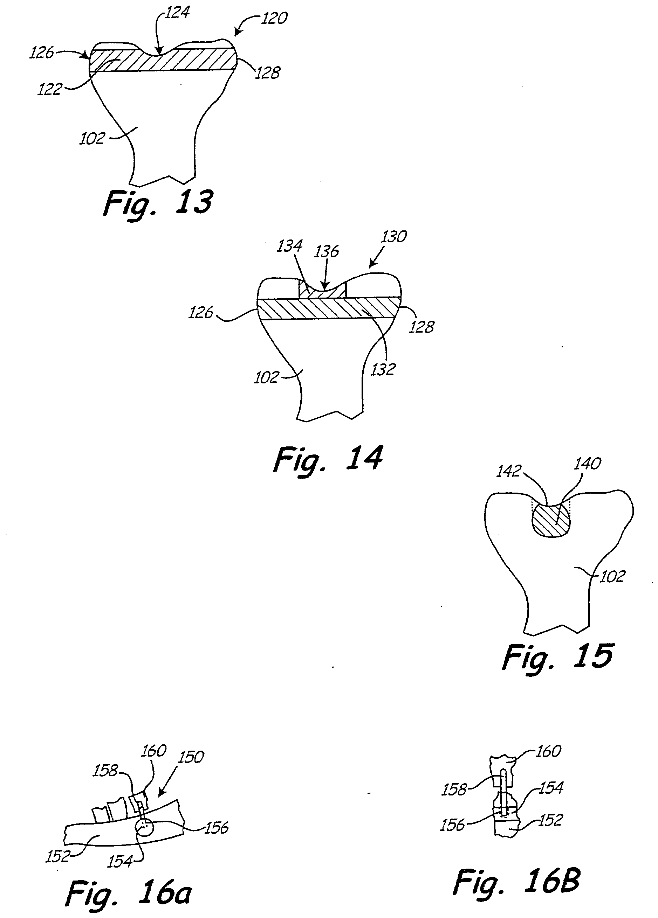

Fig. 13 zeigt sehr schematisch ein Implantat 120 mit einem zylindrischen Grundkörper 122, bei dem die Gelenkersatzfläche 124 im Peripheriebereich des Grundkörpers 122, d.h. durch Abtragung von Material im Peripheriebereich, ausgebildet ist. Die Stirnseite 126, 128 schließen mit der Peripherie des Knochens 102 im Gelenkbereich ab. Das Implantat besteht vorzugsweise aus Titanlegierung, kann aber auch aus einem anderen der genannten Knochen und/oder Gelenkersatzstoffe bestehen. Insbesondere kann die Gelenkersatzfläche 124 auf einem separat in den Grundkörper 122 einzusetzenden Teil angeordnet sein, das ein spezielles Gelenkersatzmaterial ist.

Fig. 14 zeigt ein Implantat 130 mit einem zylindrischen Grundkörper 132, das einen Adapter für ein Gelenkflächenersatzteil 134 aufweist, auf dem die Gelenkersatzfläche 136 ausgebildet ist. Als Adaptertechnik können Stifte, Schrauben Presssitz und andere an sich bekannte Techniken dienen. Der Vorteil dieser Ausbildungsform besteht darin, dass der Grundkörper 132 aus optimiertem Knochenersatzwerkstoff bestehen kann, während das Gelenkflächenersatzteil 134 für die Bildung der Gelenkfläche optimiert ist.

Fig. 15 zeigt ein kugeliges Implantat 140, das in eine kreiszylindrisches Bohrloch im Gelenkbereich des Knochens 102 eingesetzt ist. Die Gelenkersatzfläche 142 wurde durch Abtragen aus der Kugelperipherie erstellt.

Die Figuren 16a und 16b zeigen ein Implantat 150 im Kieferknochen 152. Das Implantat besteht aus einem kreiszylindrischen Grundkörper 154, der mit seiner Längsachse horizontal und quer zum Kieferknochen eingesetzt wird. Der Grundkörper 154 hat einen Adapter 156, im einfachsten Fall eine Bohrung, in der ein Stift 158 zur Aufnahme einer Krone 160 aufgenommen ist. Der Grundkörper 154 besteht vorzugsweise aus Knochenersatzmaterial mit einer guten Gewebeverträglichkeit, welches insbesondere gut in den Knochen einwachsen kann. Der Stift und die Krone können aus herkömmlichen Dentalimplantatmaterialien bestehen.

Die in den obigen Figuren dargestellten Grundkörper sind vorzugsweise kreiszylindrisch, können jedoch auch andere Formen haben, wie z.B. Kegel bzw. Konus oder Zylinder mit nicht kreisrunder Grundfläche etc.

Die in den Figuren dargestellten Beispiele sind nur zur Erläuterung des Erfindungsgegenstandes gedacht und sollen keinen schwenkenden Charakter bezüglich des Schutzbereiches mit sich bringen, der durch die nachfolgenden Ansprüche definiert wird.

Selbstverständlich ist es möglich einzelne Merkmale der Erfindung einzeln oder in einer größeren Anzahl vorzusehen, z.B. die Führungsdrähte. Wenn ausgeführt ist, dass die durch die Anordnung vorgegebene Bohrachse in etwa den Mittelpunkt der defekten Gelenkfläche schneidet, so sind Abweichungen von wenigen mm, z.B. bis zu 5 mm tolerierbar.

## Patentansprüche

1. Implantat (100, 100a, 100b) zum partiellen Ersatz von Gelenkflächen (112), bestehend aus einem zylindrischen Grundkörper (116, 122, 132) zum Einsatz in einen aus einem Knochen ausgenommenen Bereich mit einer Gelenkersatzfläche (124) zur Substitution des zu ersetzenden Gelenkflächenabschnitts, wobei der Grundkörper eine erste Stirnseite (104, 126), eine zweite Stirnseite (106, 128) und einen peripheren Bereich aufweist und wobei mindestens eine der Stirnseiten eine in einem Winkel ungleich 90° zur Zylinderachse verlaufende erste Stirnfläche aufweist, wobei die erste Stirnfläche als die Gelenkersatzfläche (104a, 104b) ausgebildet ist.

2. Implantat (100) zum partiellen Ersatz von Gelenkflächen (112), bestehend aus einem zylindrischen Grundkörper (116, 122, 132) zum Einsatz in einen aus einem Knochen ausgenommenen Bereich mit einer Gelenkersatzfläche (124) zur Substitution des zu ersetzenden Gelenkflächenabschnitts, wobei der Grundkörper eine erste Stirnseite (104, 126), eine zweite Stirnseite (106, 128) und einen peripheren Bereich aufweist und wobei eine im peripheren Bereich durch Abtragen von Material des zylindrischen Grundkörpers ausgebildete Fläche als die Gelenkersatzfläche (124) ausgebildet ist.

3. Implantat (100) nach einem der Ansprüche 1 bis 2, **dadurch gekennzeichnet, dass** der Grundkörper (116, 122, 132) kreiszylindrisch ausgebildet ist.

4. Implantat (100) nach einem der Ansprüche 1 bis 3, wobei der Grundkörper (116, 122, 132) zum Einsatz in einem Stanz- oder Bohrbereich im Knochen ausgebildet ist.

5. Implantat (100) nach Anspruch 1, 2, 3 oder 4, wobei die erste Fläche oder die im peripheren Bereich ausgebildete Gelenkersatzfläche gerade oder gekrümmt bzw. geformt ist.

6. Implantat (100) nach einem der Ansprüche 1 bis 5, **dadurch gekennzeichnet, dass** die zweite Stirnseite (106) eine zweite Fläche zum Abschluss mit der Knochenperipherie aufweist.

7. Implantat (100) nach Anspruch 6, wobei die zweite Fläche gerade oder gekrümmt bzw. geformt ist.

8. Implantat (100) nach einem der Ansprüche 1 bis 7, **dadurch gekennzeichnet, dass** es einstückig hergestellt ist.

9. Implantat (100) nach einem der vorhergehenden Ansprüche, **dadurch gekennzeichnet, dass** der Grundkörper aus einem Implantatwerkstoff oder Knochenersatzwerkstoff besteht.

10. Implantat (100) nach einem der Ansprüche 1 bis 7, **dadurch gekennzeichnet, dass** die erste und/oder zweite Stirnfläche (104, 106) oder die im peripheren Bereich ausgebildete Fläche durch ein spezielles Gelenkflächenmaterial gebildet sind.

11. Implantat (100) nach Anspruch 10, wobei das Gelenkflächenmaterial aus einem Material für künstliche Gelenke besteht, wie z.B. Keramik oder Titanlegierungen.

12. Implantat (100) nach einem der vorhergehenden Ansprüche, **dadurch gekennzeichnet, dass** der Durchmesser des Grundkörpers (116) ein wenig größer als der aus dem Knochen ausgenommene Bereich ist, so dass das Implantat im Presssitz in den aus dem Knochen ausgenommenen Bereich einsetzbar ist.

## Claims

1. An implant (100, 100a, 100b) for partially replacing joint surfaces (112), consisting of a cylindrical base body (116, 122, 132) for insertion into a region with a joint replacement surface (124) removed from a bone for substituting the joint surface section to be replaced, wherein the base body comprises a first end face (104, 126), a second end face (106, 128) and a peripheral area, and wherein at least one of the end faces comprises a first end surface extending towards the cylindrical axis at an angle not equal to 90°, wherein the first end surface is formed as the joint replacement surface (104a, 104b).

2. The implant (100) for partially replacing joint surfaces (112), consisting of a cylindrical base body (116, 122, 132) for insertion into a region with a joint replacement surface (124) removed from a bone for substituting the joint surface section to be replaced, wherein the base body comprises a first end face (104, 126), a second end face (106, 128) and a peripheral area, and wherein a surface formed by removing material of the cylindrical base body in the peripheral area is formed as the joint replacement surface (124).

3. The implant (100) according to any one of claims 1 or 2, **characterized in that** the base body (116, 122, 132) is formed circularly cylindrically.

4. The implant (100) according to any one of claims 1 to 3, wherein the base body (116, 122, 132) is formed for insertion into a punching area or drilling range in the bone.

5. The implant (100) according to any one of claims 1, 2, 3 or 4, wherein the first surface or the joint replacement surface formed in the peripheral area is straight or curved and/or shaped.

6. The implant (100) according to any one of claims 1 to 5, **characterized in that** the second end face (106) comprises a second surface to be flush with the bone periphery.

7. The implant (100) according to claim 6, wherein the second surface is straight or curved and/or shaped.

8. The Implant (100) according to any one of claims 1 to 7, **characterized in that** said implant is manufactured in one piece.

9. The implant (100) according to any one of the preceding claims, **characterized in that** the base body consists of an implant material or bone replacement material.

10. The implant (100) according to any one of claims 1 to 7, **characterized in that** the first and/or second end surface (104, 106) or the surface formed in the peripheral area is formed by a specific joint surface material.

11. The implant (100) according to claim 10, wherein the joint surface material consists of a material for artificial joints, such as, e.g., ceramic or titanium alloys.

12. The implant (100) according to any one of the preceding claims, **characterized in that** the diameter of the base body (116) is slightly larger than the region removed from the bone, so that the implant is insertable by press fit into the region removed from the bone.

## Revendications

1. Implant (100, 100a, 100b) pour le remplacement partiel de surfaces articulaires (112), constitué d'un corps de base cylindrique (116, 122, 132) destiné à être mis en place dans une zone évidée d'un os et comportant une surface articulaire de remplacement (124) destinée à se substituer au secteur de surface articulaire à remplacer, implant dans lequel le corps de base présente un premier côté frontal (104, 126), un deuxième côté frontal (106, 128) et une zone périphérique, et dans lequel au moins un des côtés frontaux présente une première surface frontale s'étendant selon un angle différent de 90° par rapport à l'axe de cylindre, et la première surface frontale est réalisée comme étant la surface articulaire de remplacement (104a, 104b).

2. Implant (100) pour le remplacement partiel de surfaces articulaires (112), constitué d'un corps de base cylindrique (116, 122, 132) destiné à être mis en place dans une zone évidée d'un os et comportant une surface articulaire de remplacement (124) destinée à se substituer au secteur de surface articulaire à remplacer, implant dans lequel le corps de base présente un premier côté frontal (104, 126), un deuxième côté frontal (106, 128) et une zone périphérique, et dans lequel une surface formée dans la zone périphérique par enlèvement de matière du corps de base cylindrique, est réalisée comme étant la surface articulaire de remplacement (124).

3. Implant (100) selon l'une des revendications 1 à 2, **caractérisé en ce que** le corps de base (116, 122, 132) est de configuration cylindrique circulaire.

4. Implant (100) selon l'une des revendications 1 à 3, dans lequel le corps de base (116, 122, 132) est configuré pour une mise en place dans une zone découpée ou percée dans l'os.

5. Implant (100) selon la revendication 1, 2, 3 ou la revendication 4, dans lequel ladite première surface ou la surface articulaire de remplacement formée dans la zone périphérique est plane ou courbe, à savoir mise en forme.

6. Implant (100) selon l'une des revendications 1 à 5, **caractérisé en ce que** ledit deuxième côté frontal (106) présente une deuxième surface pour assurer la continuité de la périphérie de l'os.

7. Implant (100) selon la revendications 6, dans lequel ladite deuxième surface est plane ou courbe, à savoir mise en forme.

8. Implant (100) selon l'une des revendications 1 à 7, **caractérisé en ce qu'**il est fabriqué d'un seul tenant.

9. Implant (100) selon l'une des revendications précédentes, **caractérisé en ce que** le corps de base est réalisé en un matériau d'implant ou en un matériau de substitution de l'os.

10. Implant (100) selon l'une des revendications 1 à 7, **caractérisé en ce que** la première et/ou la deuxième surface frontale (104, 106) ou bien la surface formée dans la zone périphérique, sont réalisées en un matériau de surface articulaire spécial.

11. Implant (100) selon la revendication 10, dans lequel le matériau de surface articulaire est constitué d'un matériau pour articulations artificielles, comme par exemple une céramique ou des alliages de titane.

12. Implant (100) selon l'une des revendications précédentes, **caractérisé en ce que** le diamètre du corps de base (116) est très légèrement plus grand que la zone évidée dans l'os, de sorte que l'implant peut être mis en place dans la zone évidée dans l'os, en y étant monté à force avec un ajustement serré.
